# EUROPÄISCHE PATENTANMELDUNG

(11) **EP 3 742 468 A1**
(43) Veröffentlichungstag der Anmeldung: **25.11.2020**
(21) Anmeldenummer: 19175359.9
(22) Anmeldetag: 20.05.2019
(51) Int. Cl.: H01J 35/06, H01J 35/08, H01J 35/30, H05G 1/30, H05G 1/32

(54) **DOSISMODULATION**

(71) Anmelder: Siemens Healthcare GmbH, 91052 Erlangen (DE)
(72) Erfinder: Fritzler, Anja, 91052 Erlangen (DE); Matuszok, Dieter, 91085 Weisendorf (DE); Petersilka, Martin, 91325 Adelsdorf (DE)

(57) **Zusammenfassung**

Die Erfindung betrifft ein Verfahren für ein Festlegen einer Intensität einer Röntgenstrahlung mit einer strukturierten Anode oder mit einer Feldemitterkathode oder mit einem fingerförmigen Kathodenkopf, eine zugehörige Röntgeneinrichtung, einen zugehörigen Single-Röntgenröhren-Computertomograph, einen zugehörigen Dual-Röntgenröhren-Computertomograph und ein zugehöriges Computerprogrammprodukt.

## Beschreibung

Die Erfindung betrifft ein Verfahren für ein Festlegen einer Intensität einer Röntgenstrahlung mit einer strukturierten Anode oder mit einer Feldemitterkathode oder mit einem fingerförmigen Kathodenkopf, eine zugehörige Röntgeneinrichtung, einen zugehörigen Single-Röntgenröhren-Computertomograph, einen zugehörigen Dual-Röntgenröhren-Computertomograph und ein zugehöriges Computerprogrammprodukt.

Eine bildgebende Messung eines Patienten mit Röntgenstrahlung kann eine Dosismodulation der Röntgenstrahlung erfordern. Die Dosismodulation entspricht üblicherweise einem Anpassen der Intensität der Röntgenstrahlung insbesondere gemäß dem Patienten während der bildgebenden Messung. Üblicherweise kann die Dosismodulation mittels einer Variation eines Heizstroms eines Emitters und/oder einer Emitterheizung erfolgen. In einer herkömmlichen Röntgenröhre ist eine Schnelligkeit der Dosismodulation insbesondere durch thermische Eigenschaften des Emitters und/oder der Emitterheizung der Röntgenröhre begrenzt. Beispielsweise kann ein herkömmlicher Flachemitter mittels des in der Emitterheizung fließenden Heizstroms durch aus der herkömmlichen Emitterheizung austretenden Heizelektronen indirekt erwärmt und damit die Emission von Elektronen aus dem herkömmlichen Flachemitter angeregt werden. Ein alternativer Flachemitter oder ein herkömmlicher Emitter wird typischerweise direkt vom Heizstrom durchströmt und dadurch zur Emission von Elektronen angeregt.

Je umfassender die Intensität der Röntgenstrahlung geändert wird, desto länger dauert dieser Vorgang üblicherweise aufgrund der vergleichsweise trägen thermischen Eigenschaften. Insbesondere eine signifikante Reduktion der Intensität der Röntgenstrahlung kann einige Zehntelsekunden in der herkömmlichen Röntgenröhre dauern. Wenn beispielsweise die herkömmliche Röntgenröhre in einem Computertomographen eingesetzt wird, kann bei einer Rotationszeit der herkömmlichen Röntgenröhre in dem Computertomographen im Sub-Sekundenbereich die Dosismodulation in diesem Fall nicht immer instantan an den Patienten angepasst werden.

Der Erfindung liegt die Aufgabe zu Grunde, ein Verfahren für ein Festlegen einer Intensität einer Röntgenstrahlung mit einer strukturierten Anode oder mit einer Feldemitterkathode oder mit einem fingerförmigen Kathodenkopf, eine zugehörige Röntgeneinrichtung, einen zugehörigen Single-Röntgenröhren-Computertomograph, einen zugehörigen Dual-Röntgenröhren-Computertomograph und ein zugehöriges Computerprogrammprodukt anzugeben, bei welchen die Intensität der Röntgenstrahlung schneller festgelegt wird.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Festlegen einer Intensität einer Röntgenstrahlung mit einer strukturierten Anode weist folgende Schritte auf:
- Bereitstellen einer Röntgenröhre, welche eine strukturierte Anode mit einer ersten Anodenmikrostruktur und einer zweiten Anodenmikrostruktur aufweist,
- Bereitstellen einer ersten Röntgenstrahlungsintensitätskennzahl gemäß der ersten Anodenmikrostruktur und einer zweiten Röntgenstrahlungsintensitätskennzahl gemäß der zweiten Anodenmikrostruktur in einer Steuereinheit,
- Ermitteln eines ersten Sollwerts der Intensität der Röntgenstrahlung in der Steuereinheit gemäß einem bei einer bildgebenden Messung zu untersuchenden Patienten,
- Auswählen der ersten Röntgenstrahlungsintensitätskennzahl oder der zweiten Röntgenstrahlungsintensitätskennzahl als erste Sollwertröntgenstrahlungsintensitätskennzahl gemäß dem ersten Sollwert der Intensität der Röntgenstrahlung in der Steuereinheit und
- Ausrichten eines Elektronenstrahls der Röntgenröhre auf die erste Anodenmikrostruktur oder auf die zweite Anodenmikrostruktur gemäß der ausgewählten ersten Sollwertröntgenstrahlungsintensitätskennzahl zur Generierung der Röntgenstrahlung, wodurch die Intensität der Röntgenstrahlung der strukturierten Anode festgelegt wird.

Das Verfahren für das Festlegen der Intensität der Röntgenstrahlung mit der strukturierten Anode bietet insbesondere folgenden Vorteil, dass die Intensität der Röntgenstrahlung insbesondere im Vergleich zur Variation des Heizstroms schnell festgelegt werden kann. Dies liegt insbesondere daran, dass der Elektronenstrahl üblicherweise schneller ausgerichtet, sprich bewegt, werden kann als ein Heizstrom eines herkömmlichen Emitters und/oder einer herkömmlichen Emitterheizung variiert werden kann und/oder als sich die Rate der Emission der Elektronen aufgrund der thermischen Eigenschaften gemäß der Variation des Heizstroms anpassen kann. Die Variation des Heizstroms ist im Vergleich zu der Ausrichtung des Elektronenstrahls üblicherweise träge. Vorteilhafterweise kann die Intensität der Röntgenstrahlung festgelegt bzw. variiert werden, obwohl der Heizstrom nicht verändert wird, wodurch eine Anzahl der emittierten Elektronen typischerweise konstant bleibt. Das erfindungsgemäße Verfahren für das Festlegen der Intensität der Röntgenstrahlung entspricht also insbesondere einer vorteilhaften Dosismodulation.

Die Dosismodulation beschreibt insbesondere ein Anpassen der Intensität der Röntgenstrahlung mit der Maßgabe, dass die Dosis für den Patienten während der bildgebenden Messung vorzugsweise fortlaufend bis zu einem Niveau verringert wird, bei welchem eine diagnostische Aussagekraft eines gemäß der Intensität der Röntgenstrahlung erfassten medizinischen Bildes sichergestellt ist. Die bildgebende Messung umfasst insbesondere ein Durchleuchten des Patienten mit der Röntgenstrahlung, um das medizinische Bild des Patienten rekonstruieren zu können. Die bildgebende Messung kann kontrastmittelgestützt und/oder dynamisch sein. Das medizinische Bild kann in einem DICOM-Bildformat nach der Rekonstruktion vorliegen. Die Dosismodulation erfolgt vorteilhafterweise während der bildgebenden Messung.

Die strukturierte Anode kann einer Stehanode oder Drehanode entsprechen. Die Drehanode kann insbesondere eine Ausführungsform einer Drehkolbenröntgenröhre und/oder einer Drehanodenröntgenröhre umfassen. Zur Generierung der Röntgenstrahlung weist die strukturierte Anode typischerweise zumindest auf einer zum Elektronenstrahl ausgerichteten Oberfläche Wolfram oder Molybdän, oder andere geeignete Materialien auf. Die Röntgenröhre ist üblicherweise vakuumisiert. Die Röntgenröhre weist typischerweise eine Kathode mit einem Emitter für ein Bereitstellen des Elektronenstrahls auf. Der Elektronenstrahl transportiert üblicherweise Elektronen vom Emitter zur strukturierten Anode im Vakuum der Röntgenröhre insbesondere in Abhängigkeit einer Beschleunigungsspannung. Der Emitter kann insbesondere ein Feldeffektemitter, ein Flachemitter oder ein Wendelemitter sein. Der Flachemitter kann insbesondere mit einer Kathode gemäß der DE 10 2008 011 841 A1 ausgebildet sein.

Die erste Anodenmikrostruktur und/oder die zweite Anodenmikrostruktur kann beispielsweise bei einer Herstellung der strukturierten Anode entstehen oder die strukturierte Anode kann nachträglich derartig bearbeitet werden. Die ersten Anodenmikrostruktur und/oder die zweite Anodenmikrostruktur beschreiben insbesondere lokale Veränderungen, beispielsweise im Mikrometerbereich, insbesondere auf der Oberfläche der strukturierten Anode. Die erste Anodenmikrostruktur kann beispielsweise mehrere lokale Veränderungen umfassen, während die zweite Anodenmikrostruktur keine lokale Veränderung umfasst. Die strukturierte Anode ist beispielsweise in der noch nicht-veröffentlichten Anmeldung EP 18196848 offenbart.

Das Bereitstellen der Röntgenröhre kann ein Herstellen der Röntgenröhre umfassen. Alternativ oder zusätzlich kann das Bereitstellen der Röntgenröhre ein Inbetriebnehmen der Röntgenröhre insbesondere für eine bildgebende Messung umfassen. Das Inbetriebnehmen kann ein Platzieren eines bei der bildgebenden Messung zu untersuchenden Patienten in der Nähe der Röntgenröhre umfassen. Das Bereitstellen der Röntgenröhre umfasst insbesondere ein Nutzen der Röntgenröhre während der bildgebenden Messung. Das Inbetriebnehmen und/oder das Nutzen kann insbesondere durch einen Nutzer und/oder einen Arzt erfolgen.

Die erste Röntgenstrahlungsintensitätskennzahl sowie die zweite Röntgenstrahlungsintensitätskennzahl beschreiben insbesondere eine Effizienz beim Umwandeln von den auf der jeweiligen Anodenmikrostruktur der strukturierten Anode auftreffenden Elektronen des Elektronenstrahls in Röntgenstrahlung. Die erste Röntgenstrahlungsintensitätskennzahl und/oder die zweite Röntgenstrahlungsintensitätskennzahl können jeweils eine Röntgenstrahlungsintensitätskennlinie umfassen. Die erste Röntgenstrahlungsintensitätskennzahl sowie die zweite Röntgenstrahlungsintensitätskennzahl korrelieren insbesondere mit einer Dosis der Röntgenstrahlung und/oder unterscheiden sich vorzugsweise derart, dass eine Dosismodulation ermöglicht wird. Typischerweise unterscheiden sich die erste Röntgenstrahlungsintensitätskennzahl sowie die zweite Röntgenstrahlungsintensitätskennzahl derart, dass die Intensität der Röntgenstrahlung vorzugsweise von der ausgewählten Anodenmikrostruktur abhängt. Vorteilhafterweise sind die erste Röntgenstrahlungsintensitätskennzahl und die zweite Röntgenstrahlungsintensitätskennzahl unabhängig von einer Intensität des Elektronenstrahls. Üblicherweise wird für jede Anodenmikrostruktur eine separate Röntgenstrahlungsintensitätskennzahl bereitgestellt. Beispielsweise kann die Intensität der Röntgenstrahlung desto stärker sein, je höher die Röntgenstrahlungsintensitätskennzahl ist.

Die Röntgenstrahlungsintensitätskennzahl kann insbesondere von der Beschleunigungsspannung abhängig sein. Die Beschleunigungsspannung kann die erste Röntgenstrahlungsintensitätskennzahl und die zweite Röntgenstrahlungsintensitätskennzahl insbesondere derart beeinflussen, dass ein Verhältnis der ersten Röntgenstrahlungsintensitätskennzahl und der zweiten Röntgenstrahlungsintensitätskennzahl ebenfalls abhängig von der Beschleunigungsspannung ist. Beispielsweise kann das Verhältnis bei einer Beschleunigungsspannung von 120 kV 60/100 oder bei 80 kV 70/90 relativ zu einem Maximalwert sein. Die Röntgenstrahlungsintensitätskennzahl hängt insbesondere von einer Form der Anodenmikrostruktur ab. Die Röntgenstrahlungsintensitätskennzahl hängt typischerweise von einer freien Weglänge und/oder einem Streukoeffizienten des Elektronenstrahls in der Anodenmikrostruktur ab.

Das Bereitstellen der Röntgenstrahlungsintensitätskennzahl kann insbesondere ein Abrufen der ersten Röntgenstrahlungsintensitätskennzahl und/oder ein Abrufen der zweiten Röntgenstrahlungsintensitätskennzahl aus einer Speichereinheit umfassen. Grundsätzlich ist es denkbar, dass die erste Röntgenstrahlungsintensitätskennzahl sowie die zweite Röntgenstrahlungsintensitätskennzahl von dem Hersteller der Röntgenröhre, insbesondere der strukturierten Anode, ermittelt und in der Speichereinheit abgespeichert wird. Vorteilhafterweise können die erste Röntgenstrahlungsintensitätskennzahl und/oder die zweite Röntgenstrahlungsintensitätskennzahl beispielsweise durch den Nutzer und/oder den Arzt bei einer Referenzmessung ermittelt werden. Die Referenzmessung kann eine weitere bildgebende Messung eines Phantoms und/oder eines weiteren Patienten umfassen. Das Bereitstellen der Röntgenstrahlungsintensitätskennzahl kann ein Modellieren oder ein Simulieren der strukturierten Anode umfassen. Die Speichereinheit kann Teil der Röntgenröhre sein. Das Bereitstellen umfasst insbesondere ein Bereitstellen der ersten Röntgenstrahlungsintensitätskennzahl sowie der zweiten Röntgenstrahlungsintensitätskennzahl als Eingangsparameter für das Auswählen der ersten Röntgenstrahlungsintensitätskennzahl oder der zweiten Röntgenstrahlungsintensitätskennzahl.

Der erste Sollwert der Intensität der Röntgenstrahlung beschreibt insbesondere einen vorgegebenen Zielwert der Intensität der Röntgenstrahlung nach dem Ausrichten des Elektronenstrahls. Der erste Sollwert gibt insbesondere die Intensität der Röntgenstrahlung vor. Der erste Sollwert kann insbesondere von einer Anatomie und/oder einer Physiologie des Patienten abhängen. Beispielsweise kann der erste Sollwert gemäß einem EKG-Signal der Physiologie des Patienten abhängen. Alternativ oder zusätzlich kann der erste Sollwert von einer Ausdehnung des Patienten oder einer anatomischen Struktur des Patienten entlang der Röntgenstrahlungsrichtung abhängen. Die anatomische Struktur beschreibt insbesondere ein Organ und/oder einen Knochen und/oder ein Gefäß. Typischerweise ist der erste Sollwert desto höher, je röntgendichter die anatomische Struktur oder der Patient in Abhängigkeit von der Ausrichtung der Röntgenstrahlung ist. Beispielsweise ist der Patient bei einer Durchleuchtung parallel zu einer Schulter des Patienten röntgendichter als bei einer frontalen Durchleuchtung eines Abdomens des Patienten.

Die Steuereinheit ist insbesondere zum Ermitteln des ersten Sollwerts, insbesondere gemäß einem Sollwertalgorithmus, ausgebildet. Beispielsweise kann die Steuereinheit ein Signal eines physiologischen Messsystems beim Ermitteln des ersten Sollwerts berücksichtigen. Das physiologische Messsystem kann vorteilhafterweise die Physiologie des Patienten, insbesondere das EKG-Signal und/oder eine Atmung, erfassen. Alternativ oder zusätzlich kann die Steuereinheit beispielsweise mittels eines medizinischen Übersichtsbildes den ersten Sollwert der Intensität ermitteln. Das medizinische Übersichtsbild kann einem Lokalizer-Bild oder einem älteren medizinischen Bild des Patienten entsprechen und/oder stellt vorzugsweise die Anatomie des Patienten dar. Grundsätzlich ist es denkbar, dass der erste Sollwert beispielsweise von einem Rotationswinkel der Röntgenröhre abhängt. Das Ermitteln des ersten Sollwerts kann unter einer Maßgabe einer Dosisreduktion für den Patienten erfolgen.

Das Auswählen der ersten Röntgenstrahlungsintensitätskennzahl oder der zweiten Röntgenstrahlungsintensitätskennzahl umfasst insbesondere ein Vergleichen des ersten Sollwerts der Intensität der Röntgenstrahlung mit der ersten Röntgenstrahlungsintensitätskennzahl und der zweiten Röntgenstrahlungsintensitätskennzahl, wobei beispielsweise zwei Differenzwerte errechnet und der betragsmäßig geringere Differenzwert ausgewählt wird. Die erste Sollwertröntgenstrahlungsintensitätskennzahl ist vorzugsweise durch den betragsmäßig geringeren Differenzwert identifizierbar. In anderen Worten wird vorzugsweise die Röntgenstrahlungsintensitätskennzahl ausgewählt, welcher dem Sollwert der Intensität der Röntgenstrahlung vorzugsweise betragsmäßig am nächsten kommt, besonders vorteilhafterweise entspricht. Wenn die Differenzwerte identisch sind, kann beispielsweise diejenige Röntgenstrahlungsintensitätskennzahl der vormals nicht bestrahlten Anodenmikrostruktur ausgewählt werden, um eine gleichmäßige Wärmeverteilung zu gewährleisten. Die erste Sollwertröntgenstrahlungsintensitätskennzahl entspricht typischerweise der ausgewählten ersten Röntgenstrahlungsintensitätskennzahl oder der ausgewählten zweiten Röntgenstrahlungsintensitätskennzahl. Typischerweise ist die Steuereinheit zum Auswählen der Sollwertröntgenstrahlungsintensitätskennzahl ausgebildet.

Die Steuereinheit ist insbesondere mit einer Ablenkeinheit der Röntgenröhre zum Ausrichten des Elektronenstrahls verbunden. Das Ausrichten des Elektronenstrahls beschreibt insbesondere ein Festlegen eines Fokuspunkts des Elektronenstrahls auf der strukturierten Anode. Der Fokuspunkt überschneidet sich vorteilhafterweise mit der ersten Anodenmikrostruktur und/oder der zweiten Anodenmikrostruktur. Der Fokuspunkt umfasst insbesondere die erste Anodenmikrostruktur und/oder die zweite Anodenmikrostruktur. Der Elektronenstrahl kann typischerweise wahlweise auf die erste Anodenmikrostruktur oder auf die zweite Anodenmikrostruktur ausgerichtet werden, wodurch die Elektronen vorzugsweise auf der ersten Anodenmikrostruktur oder auf der zweiten Anodenmikrostruktur auftreffen. Grundsätzlich ist es denkbar, dass eine Ortsverteilung des Elektronenstrahls derart gewählt wird, dass der Elektronenstrahl die erste Anodenmikrostruktur oder die zweite Anodenmikrostruktur zumindest teilweise oder vollständig oder die erste Anodenmikrostruktur und die zweite Anodenmikrostruktur zusammen zumindest teilweise oder vollständig umfasst. Der Elektronenstrahl kann einen Bereich auf der strukturierten Anode außerhalb der ersten Anodenmikrostruktur und/oder der zweiten Anodenmikrostruktur treffen. Das Ausrichten des Elektronenstrahls mittels der Ablenkeinheit ist vorzugsweise schnell im Vergleich zur Variation des Heizstroms.

Typischerweise wird beim Auftreffen der Elektronen des Elektronenstrahls die Röntgenstrahlung mit einer Intensität gemäß der jeweiligen Röntgenstrahlungsintensitätskennzahl generiert. Durch das Ausrichten des Elektronenstrahls wird die Intensität der Röntgenstrahlung insbesondere festgelegt, weil die Intensität der Röntgenstrahlung typischerweise mit der jeweiligen Röntgenstrahlungsintensitätskennzahl korreliert.

Eine Ausführungsform sieht vor, dass ein zweiter Sollwert der Intensität der Röntgenstrahlung in der Steuereinheit gemäß dem bei der bildgebenden Messung zu untersuchenden Patienten ermittelt wird, wobei eine zweite Sollwertröntgenstrahlungsintensitätskennzahl gemäß dem zweiten Sollwert der Intensität der Röntgenstrahlung in der Steuereinheit ausgewählt wird und wobei der Elektronenstrahl der Röntgenröhre auf die erste Anodenmikrostruktur oder auf die zweite Anodenmikrostruktur gemäß der ausgewählten zweiten Sollwertröntgenstrahlungsintensitätskennzahl erneut ausgerichtet wird. Diese Ausführungsform ist insofern vorteilhaft, weil die Intensität der Röntgenstrahlung durch das erneute Festlegen geändert werden kann. Insbesondere wenn mehrere medizinische Bilder und/oder mehrere Röntgenprojektionen in Abhängigkeit von der generierten Röntgenstrahlung während der bildgebenden Messung erfasst werden sollen, ist diese Ausführungsform vorteilhaft, weil für jedes medizinisches Bild und/oder jede Röntgenprojektion die Intensität der Röntgenstrahlung angepasst werden kann. Das erneute Ausrichten des Elektronenstrahls gemäß der ausgewählten zweiten Sollwertröntgenstrahlungsintensitätskennzahl erfolgt typischerweise zeitlich nach dem Ausrichten des Elektronenstrahls gemäß der ausgewählten ersten Sollwertröntgenstrahlungsintensitätskennzahl. Das Ermitteln des zweiten Sollwerts kann beim Ermitteln des ersten Sollwerts oder danach erfolgen. Die zweite Sollwertröntgenstrahlungsintensitätskennzahl entspricht typischerweise der ausgewählten ersten Röntgenstrahlungsintensitätskennzahl oder der ausgewählten zweiten Röntgenstrahlungsintensitätskennzahl. Grundsätzlich ist es denkbar, dass der Elektronenstrahl mehrmals ausgerichtet wird oder unverändert bleibt.

Eine Ausführungsform sieht vor, dass das erneute Ausrichten des Elektronenstrahls gemäß der ausgewählten zweiten Sollwertröntgenstrahlungsintensitätskennzahl innerhalb einer Elektronenstrahlausrichtungszeit von weniger als 1 s nach dem Ausrichten des Elektronenstrahls gemäß der ausgewählten ersten Sollwertröntgenstrahlungsintensitätskennzahl erfolgt. Diese Ausführungsform ermöglicht typischerweise das erneute Ausrichten während derselben bildgebenden Messung.

Eine Ausführungsform sieht vor, dass die Elektronenstrahlausrichtungszeit geringer ist als 1 ms. Die Elektronenstrahlausrichtungszeit kann insbesondere zwischen 1 bis 900 µs, vorzugsweise zwischen 10 bis 100 µs liegen. Diese Ausführungsform ist insbesondere vorteilhaft, weil die Elektronenstrahlausrichtungszeit in derselben Größenordnung liegt wie eine Röntgendetektorauslesezeit. Die Röntgendetektorauslesezeit ist beispielsweise geringer als 1 ms bzw. liegt insbesondere zwischen 100 bis 300 µs.

Eine Ausführungsform sieht vor, dass die Elektronenstrahlausrichtungszeit auf die Röntgendetektorauslesezeit der bildgebenden Messung abgestimmt wird. In dieser Ausführungsform kann beispielsweise der Elektronenstrahl im Gleichtakt zum Auslesen des Röntgendetektors ausgerichtet werden. Diese Ausführungsform bietet daher insbesondere Vorteile, wenn die mehreren medizinischen Bilder und/oder die mehreren Röntgenprojektionen erfasst werden. Typischerweise dauert nämlich das Erfassen einer einzelnen Röntgenprojektion so lang wie die Röntgendetektorauslesezeit. Das medizinische Bild weist typischerweise eine Vielzahl an Röntgenprojektionen auf. Dementsprechend dauert das Erfassen eines einzelnen medizinisches Bildes typischerweise mehrere Röntgendetektorauslesezeiten lang.

Eine Ausführungsform sieht vor, dass sich die erste Anodenmikrostruktur von der zweiten Anodenmikrostruktur in zumindest einem der folgenden Parameter unterscheidet:
- einer Rillentiefe,
- einer Rillenbreite,
- einem Mittelpunktabstand,
- in einer Punktierung einer Rille. Diese Ausführungsform beschreibt insbesondere einfach umzusetzende lokale Veränderungen auf der Oberfläche der strukturierten Anode, insbesondere der ersten Anodenmikrostruktur und der zweiten Anodenmikrostruktur zu gestalten. Die lokalen Veränderungen auf der Oberfläche können insbesondere die Form der Anodenmikrostruktur beschreiben. Die Rillentiefe liegt beispielsweise im Bereich zwischen 0,1 und 1000 µm, vorzugsweise zwischen 1 und 100 µm, besonders vorteilhafterweise zwischen 10 bis 15 µm. Die Rillenbreite liegt beispielsweise im Bereich zwischen 0,1 und 1000 µm, vorzugsweise zwischen 1 und 100 µm, besonders vorteilhafterweise zwischen 10 bis 15 µm. Der Unterschied im Mittelpunktabstand ist insbesondere in Kombination mit einem verschiedenen Einfallwinkel des Elektronenstrahls vorteilhaft. Der Mittelpunktabstand beschreibt insbesondere einen Abstand zu einem Mittelpunkt oder einem Referenzpunkt der strukturierten Anode. Wenn die strukturierte Anode der Drehanode entspricht, ist beispielsweise der Mittelpunkt ein Drehachsenpunkt der Drehanode. Wenn die strukturierte Anode alternativ der Stehanode entspricht, entspricht der Referenzpunkt beispielsweise einem Punkt auf einer Gerade definiert durch die erste Anodenmikrostruktur und die zweite Anodenmikrostruktur, wobei der Punkt nicht zwischen der ersten Anodenmikrostruktur und der zweiten Anodenmikrostruktur liegt und dadurch typischerweise der Einfallwinkel des Elektronenstrahls variiert. Typischerweise ist die Effizienz der Generierung der Röntgenstrahlung desto geringer, je stärker die Punktierung ausgeprägt ist. Die Punktierung ermöglicht daher insbesondere ein einfaches Anpassen der Intensität der Röntgenstrahlung. Die Punktierung kann beispielsweise 1 bis 50%, vorteilhafterweise zwischen 10 bis 20% der Rillenbreite oder einer Rillenlänge umfassen. Beispielsweise kann die erste Anodenmikrostruktur eben sein und die zweite Anodenmikrostruktur eine und/oder mehrere Rillen aufweisen. Jede Rille kann dieselbe Rillenbreite oder dieselbe Rillentiefe oder alternative verschiedene Rillenbreite und/oder verschiedene Rillentiefen aufweisen. Eine Anzahl der Rillen der ersten Anodenmikrostruktur kann einer Anzahl der Rillen der zweiten Anodenmikrostruktur entsprechen.

Eine Ausführungsform sieht vor, dass der Elektronenstrahl mittels einer elektromagnetischen Ablenkeinheit oder mittels eines Wehneltzylinders ausgerichtet wird. Die elektromagnetische Ablenkeinheit kann insbesondere einen magnetischen Quadrupol umfassen. In diesem Fall weist die Röntgenröhre die elektromagnetische Ablenkeinheit oder den Wehneltzylinder vorteilhafterweise auf. Diese Ausführungsform ist insbesondere vorteilhaft, weil eine schnelle Auslenkung des Elektronenstrahls gemäß der Elektronenstrahlausrichtungszeit ermöglicht wird. Die elektromagnetische Ablenkeinheit oder der Wehneltzylinder sind insbesondere mit der Steuereinheit verbunden.

Eine Ausführungsform sieht vor, dass eine weitere strukturierte Anode mit einer dritten Anodenmikrostruktur und einer vierten Anodenmikrostruktur als Teil einer weiteren Röntgenröhre bereitgestellt wird, wobei eine dritte Röntgenstrahlungsintensitätskennzahl gemäß der dritten Anodenmikrostruktur und eine vierte Röntgenstrahlungsintensitätskennzahl gemäß der vierten Anodenmikrostruktur in der Steuereinheit bereitgestellt wird, wobei ein weiterer Sollwert einer Intensität einer Röntgenstrahlung der weiteren strukturierten Anode in der Steuereinheit komplementär zum ersten Sollwert ermittelt wird, wobei die dritte Röntgenstrahlungsintensitätskennzahl oder die vierte Röntgenstrahlungsintensitätskennzahl als weitere Sollwertröntgenstrahlungsintensitätskennzahl gemäß dem weiteren Sollwert der Intensität der Röntgenstrahlung in der Steuereinheit ausgewählt wird und wobei ein weiterer Elektronenstrahl der weiteren Röntgenröhre auf die dritte Anodenmikrostruktur oder auf die vierte Anodenmikrostruktur gemäß der ausgewählten weiteren Sollwertröntgenstrahlungsintensitätskennzahl zur Generierung der Röntgenstrahlung ausgerichtet wird, wodurch die Intensität der Röntgenstrahlung der weiteren strukturierten Anode festgelegt wird. Diese Ausführungsform beschreibt insbesondere den Betrieb der weiteren Röntgenröhre im Gegentakt zur Röntgenröhre aufgrund des komplementären Ermittelns des weiteren Sollwerts. Im Wesentlichen sind daher die Röntgenröhre und die weitere Röntgenröhre vorzugsweise baugleich und/oder werden insbesondere synchron betrieben. Die Röntgenröhre und die weitere Röntgenröhre sind insbesondere separat zum Festlegen der Intensität der Röntgenstrahlung, beispielsweise in der Steuereinheit, ausgebildet. Diese Ausführungsform kennzeichnet insbesondere, dass der weitere Sollwert der Intensität der Röntgenstrahlung der weiteren strukturierten Anode in der Steuereinheit komplementär zum ersten Sollwert ermittelt wird. Das komplementäre Ermitteln bedeutet insbesondere, dass die Intensität der Röntgenstrahlung der Röntgenröhre verringert wird, wenn die Intensität der Röntgenstrahlung der weiteren Röntgenröhre erhöht wird. Dadurch kann vorteilhafterweise ein Streustrahlen-zu-Primärstrahlen-Verhältnis der Röntgenröhre und der weiteren Röntgenröhre positiv beeinflusst werden, weil der Gegentakt die Menge an Streustrahlen verringert, insbesondere ohne den Heizstrom zu verändern. Dadurch bleibt eine Leistungsreserve der sich im Gegentakt befindenden Röntgenröhre erhalten, weil der Elektronenstrahl lediglich auf eine vergleichsweise Intensitätsärmere Anodenmikrostruktur ausgerichtet wird, um die Intensität der Röntgenstrahlung zu verringern, ohne den Heizstrom herunterregeln zu müssen.

Die erfindungsgemäße Röntgeneinrichtung weist insbesondere die Röntgenröhre und die Steuereinheit auf. Die Steuereinheit ist typischerweise mit der Röntgenröhre, insbesondere mit der Ablenkeinheit, für ein Ausrichten des Elektronenstrahls verbunden. Die Röntgeneinrichtung weist einen Röntgendetektor mit der Röntgendetektorauslesezeit auf. Vorteilhafterweise sind die Röntgenröhre und der Röntgendetektor mittels der Steuereinheit aufeinander während der bildgebenden Messung abgestimmt. Die Röntgenröhre kann insbesondere die elektromagnetische Ablenkeinheit oder den Wehneltzylinder als Ablenkeinheit aufweisen.

Der erfindungsgemäße Single-Röntgenröhren-Computertomograph weist insbesondere die Röntgeneinrichtung auf. In diesem Fall rotiert typischerweise die Röntgeneinrichtung zumindest teilweise um den Patienten. Insbesondere in diesem Fall kann der erste Sollwert von dem Rotationswinkel der Röntgenröhre abhängen.

Die Röntgeneinrichtung des erfindungsgemäßen Dual-Röntgenröhren-Computertomographen weist typischerweise zusätzlich zur Röntgenröhre die weitere Röntgenröhre auf. Die Röntgenröhre und die weitere Röntgenröhre können grundsätzlich baugleich aufgebaut sein und dennoch im Gegentakt betrieben werden. Dies ist insbesondere vorteilhaft, wenn die Intensität der beiden Röntgenröhren im Gegentakt angepasst wird, um das Streustrahlen-zu-Primärstrahlen-Verhältnis zu optimieren.

Das erfindungsgemäße Verfahren für ein Festlegen einer Intensität einer Röntgenstrahlung mit einer Feldemitterkathode weist folgende Schritte auf:
- Bereitstellen einer Feldemitterkathode,
- Bereitstellen einer Röntgenstrahlungsintensitätskennlinie als Funktion einer Emitterspannung an der Feldemitterkathode in einer Steuereinheit,
- Ermitteln eines Sollwerts der Intensität der Röntgenstrahlung gemäß einem bei einer bildgebenden Messung zu untersuchenden Patienten in der Steuereinheit,
- Auswählen der Emitterspannung aus der Röntgenstrahlungsintensitätskennlinie gemäß dem Sollwert der Intensität der Röntgenstrahlung und
- Anlegen der ausgewählten Emitterspannung an der Feldemitterkathode, wodurch die Intensität der Röntgenstrahlung festgelegt wird.

Die Feldemitterkathode weist typischerweise Feldemitterkathodennanoröhrchen auf, welche beispielsweise Silizium oder Kohlenstoff aufweisen. Der Elektronenstrahl der Feldemitterkathode wird typischerweise derart generiert, dass eine vergleichsweise geringe Emitterspannung (40 bis 100 V) an der Feldemitterkathode angelegt wird, wodurch ein starkes elektrisches Feld innerhalb der Feldemitterkathode, insbesondere zwischen den Feldemitterkathodennanoröhrchen, entsteht, wobei die Elektronen emittiert werden. Dieses Verfahren ist insofern vorteilhaft, weil die vergleichsweise geringe Emitterspannung schnell angelegt oder abgeschaltet werden kann. Im Vergleich zum Verfahren für das Festlegen der Intensität der Röntgenstrahlung mit der strukturierten Anode wird üblicherweise nicht der Elektronenstrahl ausgerichtet, sondern die Intensität des Elektronenstrahls angepasst. Dies kann insbesondere vorteilhaft sein, weil die Regelung der Intensität der Elektronenstrahls entkoppelt ist von der Fokussierung des Elektronenstrahls.

Das erfindungsgemäße Verfahren für ein Festlegen einer Intensität einer Röntgenstrahlung mit einem fingerförmigen Kathodenkopf weist folgende Schritte auf:
- Bereitstellen eines mäanderförmigen Flachemitters mit einem fingerförmigen Kathodenkopf,
- Bereitstellen einer Röntgenstrahlungsintensitätskennlinie als Funktion einer Sperrspannung an dem fingerförmigen Kathodenkopf in einer Steuereinheit,
- Ermitteln eines Sollwerts der Intensität der Röntgenstrahlung gemäß einem bei einer bildgebenden Messung zu untersuchenden Patienten in der Steuereinheit,
- Auswählen der Sperrspannung aus der Röntgenstrahlungsintensitätskennlinie gemäß dem Sollwert der Intensität der Röntgenstrahlung und
- Anlegen der ausgewählten Sperrspannung an dem fingerförmigen Kathodenkopf, wodurch die Intensität der Röntgenstrahlung festgelegt wird.

Insbesondere wenn der mäanderförmige Flachemitter gemäß der DE 10 2008 011 841 A1 ausgebildet ist, kann mittels der an dem Kathodenkopf angelegten Sperrspannung, welche beispielsweise zwischen 10 V und 5 kV, vorteilhafterweise zwischen 100 V und 2 kV liegt, die Emission der Elektronen schnell unterdrückt werden. Ein Anlegen einer Sperrspannung an einem herkömmlichen Kathodenkopf mit einem gewölbten Emitter ist beispielsweise in der DE 10 2012 211 285 B3 offenbart. Diese Ausführungsform ist insbesondere vorteilhaft, weil der mäanderförmige Flachemitter mit dem fingerförmigen Kathodenkopf bei vergleichsweise geringen Sperrspannungen gesperrt werden kann. Aufgrund der betragsmäßig geringen Sperrspannung wird vorzugsweise der Elektronenstrahl, insbesondere die Ausdehnung des Elektronenstrahls, konstant gehalten. Beispielsweise ermöglicht die Ausführungsform unabhängig von der Sperrspannung eine parallel Elektronenstrahlemission. Grundsätzlich ist es denkbar, dass nach dem Anlegen der ausgewählten Sperrspannung die vorherige Ausdehnung des Elektronenstrahls mittels der Ablenkeinheit wiederhergestellt wird. Diese Ausführungsform ist insbesondere in Hinblick auf eine Bildqualität vorteilhaft. Das Auswählen der Sperrspannung kann insbesondere ein partielles Sperren und/oder ein vollständiges Sperren des Elektronenstrahls umfassen. Das partielle Sperren des Elektronenstrahls kann insbesondere einem Dimmen des Elektronenstrahls entsprechen.

Das erfindungsgemäße Computerprogrammprodukt ist direkt in einen Speicher einer Recheneinheit ladbar und weist Programmcodemittel auf, um ein Verfahren für ein Festlegen einer Intensität einer Röntgenstrahlung auszuführen, wenn das Computerprogrammprodukt in der Recheneinheit ausgeführt wird. Die Steuereinheit kann die Recheneinheit aufweisen.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Flussdiagramm eines Verfahrens für ein Festlegen einer Intensität einer Röntgenstrahlung mit einer strukturierten Anode in einem ersten Ausführungsbeispiel,
Fig. 2 ein Flussdiagramm des Verfahrens für ein Festlegen einer Intensität einer Röntgenstrahlung mit einer strukturierten Anode in einem zweiten Ausführungsbeispiel,
Fig. 3 ein Flussdiagramm eines Verfahrens für ein Festlegen einer Intensität einer Röntgenstrahlung mit einer Feldemitterkathode in einem dritten Ausführungsbeispiel,
Fig. 4 ein Flussdiagramm eines Verfahrens für ein Festlegen einer Intensität einer Röntgenstrahlung mit einem fingerförmigen Kathodenkopf in einem vierten,
Fig. 5 eine Röntgeneinrichtung und
Fig. 6 die Röntgenröhre.

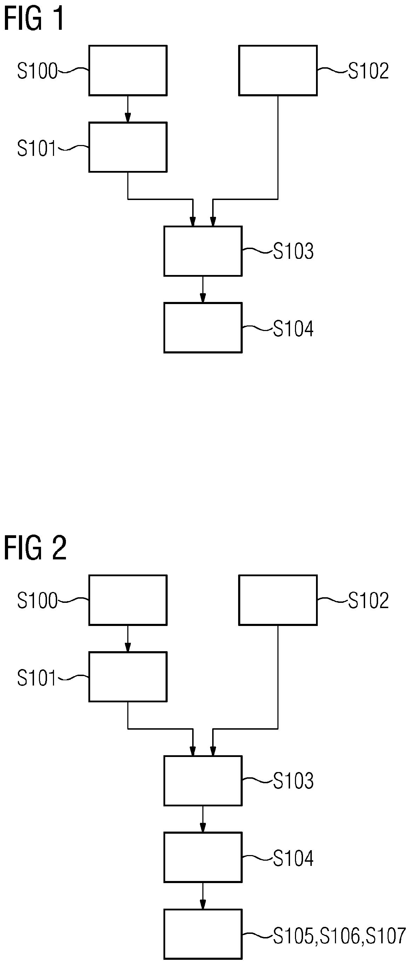

**Fig. 1** zeigt ein Flussdiagramm eines Verfahrens für ein Festlegen einer Intensität einer Röntgenstrahlung mit einer strukturierten Anode in einem ersten Ausführungsbeispiel.

Verfahrensschritt S100 kennzeichnet ein Bereitstellen einer Röntgenröhre, welche eine strukturierte Anode mit einer ersten Anodenmikrostruktur und einer zweiten Anodenmikrostruktur aufweist.

Verfahrensschritt S101 kennzeichnet ein Bereitstellen einer ersten Röntgenstrahlungsintensitätskennzahl gemäß der ersten Anodenmikrostruktur und einer zweiten Röntgenstrahlungsintensitätskennzahl gemäß der zweiten Anodenmikrostruktur in einer Steuereinheit.

Verfahrensschritt S102 kennzeichnet ein Ermitteln eines ersten Sollwerts der Intensität der Röntgenstrahlung in der Steuereinheit gemäß einem bei einer bildgebenden Messung zu untersuchenden Patienten.

Verfahrensschritt S103 kennzeichnet ein Auswählen der ersten Röntgenstrahlungsintensitätskennzahl oder der zweiten Röntgenstrahlungsintensitätskennzahl als erste Sollwertröntgenstrahlungsintensitätskennzahl gemäß dem ersten Sollwert der Intensität der Röntgenstrahlung in der Steuereinheit.

Verfahrensschritt S104 kennzeichnet ein Ausrichten eines Elektronenstrahls der Röntgenröhre auf die erste Anodenmikrostruktur oder auf die zweite Anodenmikrostruktur gemäß der ausgewählten ersten Sollwertröntgenstrahlungsintensitätskennzahl zur Generierung der Röntgenstrahlung, wodurch die Intensität der Röntgenstrahlung der strukturierten Anode festgelegt wird.

**Fig. 2** zeigt insbesondere das erste Ausführungsbeispiel gemäß Fig. 1 ergänzt um die weiteren Verfahrensschritten S105 bis S107, woraus sich das zweite Ausführungsbeispiel ergibt.

Verfahrensschritt S105 kennzeichnet, dass ein zweiter Sollwert der Intensität der Röntgenstrahlung in der Steuereinheit gemäß dem bei der bildgebenden Messung zu untersuchenden Patienten ermittelt wird, wobei eine zweite Sollwertröntgenstrahlungsintensitätskennzahl gemäß dem zweiten Sollwert der Intensität der Röntgenstrahlung in der Steuereinheit ausgewählt wird und wobei der Elektronenstrahl der Röntgenröhre auf die erste Anodenmikrostruktur oder auf die zweite Anodenmikrostruktur gemäß der ausgewählten zweiten Sollwertröntgenstrahlungsintensitätskennzahl erneut ausgerichtet wird.

Verfahrensschritt S106 kennzeichnet, dass das erneute Ausrichten des Elektronenstrahls gemäß der ausgewählten zweiten Sollwertröntgenstrahlungsintensitätskennzahl innerhalb einer Elektronenstrahlausrichtungszeit von weniger als 1 s nach dem Ausrichten des Elektronenstrahls gemäß der ausgewählten ersten Sollwertröntgenstrahlungsintensitätskennzahl erfolgt. In einem besonders vorteilhaften Ausführungsbeispiel ist die Elektronenstrahlausrichtungszeit geringer als 1 ms.

Verfahrensschritt S107 kennzeichnet, dass die Elektronenstrahlausrichtungszeit auf eine Röntgendetektorauslesezeit der bildgebenden Messung abgestimmt wird.

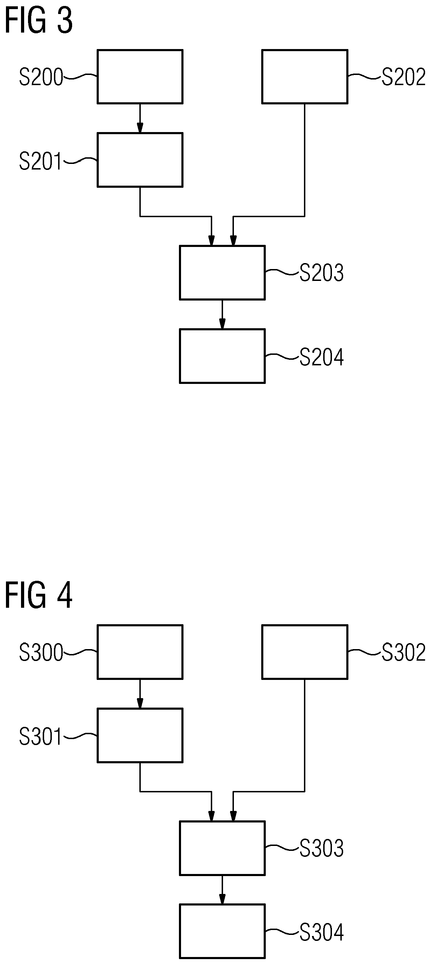

**Fig. 3** zeigt ein Flussdiagramm eines Verfahrens für ein Festlegen einer Intensität einer Röntgenstrahlung mit einer Feldemitterkathode in einem dritten Ausführungsbeispiel.

Verfahrensschritt S200 kennzeichnet ein Bereitstellen einer Feldemitterkathode.

Verfahrensschritt S201 kennzeichnet ein Bereitstellen einer Röntgenstrahlungsintensitätskennlinie als Funktion einer Emitterspannung an der Feldemitterkathode in einer Steuereinheit.

Verfahrensschritt S202 kennzeichnet ein Ermitteln eines Sollwerts der Intensität der Röntgenstrahlung gemäß einem bei einer bildgebenden Messung zu untersuchenden Patienten in der Steuereinheit.

Verfahrensschritt S203 kennzeichnet ein Auswählen der Emitterspannung aus der Röntgenstrahlungsintensitätskennlinie gemäß dem Sollwert der Intensität der Röntgenstrahlung.

Verfahrensschritt S204 kennzeichnet ein Anlegen der ausgewählten Emitterspannung an der Feldemitterkathode, wodurch die Intensität der Röntgenstrahlung festgelegt wird.

**Fig. 4** zeigt ein Flussdiagramm eines Verfahrens für ein Festlegen einer Intensität einer Röntgenstrahlung mit einem fingerförmigen Kathodenkopf in einem vierten Ausführungsbeispiel.

Verfahrensschritt S300 kennzeichnet ein Bereitstellen eines mäanderförmigen Emitters mit einem fingerförmigen Kathodenkopf .

Verfahrensschritt S301 kennzeichnet ein Bereitstellen einer Röntgenstrahlungsintensitätskennlinie als Funktion einer Sperrspannung an dem fingerförmigen Kathodenkopf in einer Steuereinheit.

Verfahrensschritt S302 kennzeichnet ein Ermitteln eines Sollwerts der Intensität der Röntgenstrahlung gemäß einem bei einer bildgebenden Messung zu untersuchenden Patienten in der Steuereinheit.

Verfahrensschritt S303 kennzeichnet ein Auswählen der Sperrspannung aus der Röntgenstrahlungsintensitätskennlinie gemäß dem Sollwert der Intensität der Röntgenstrahlung. Verfahrensschritt S304 kennzeichnet ein Anlegen der ausgewählten Sperrspannung an dem fingerförmigen Kathodenkopf, wodurch die Intensität der Röntgenstrahlung festgelegt wird.

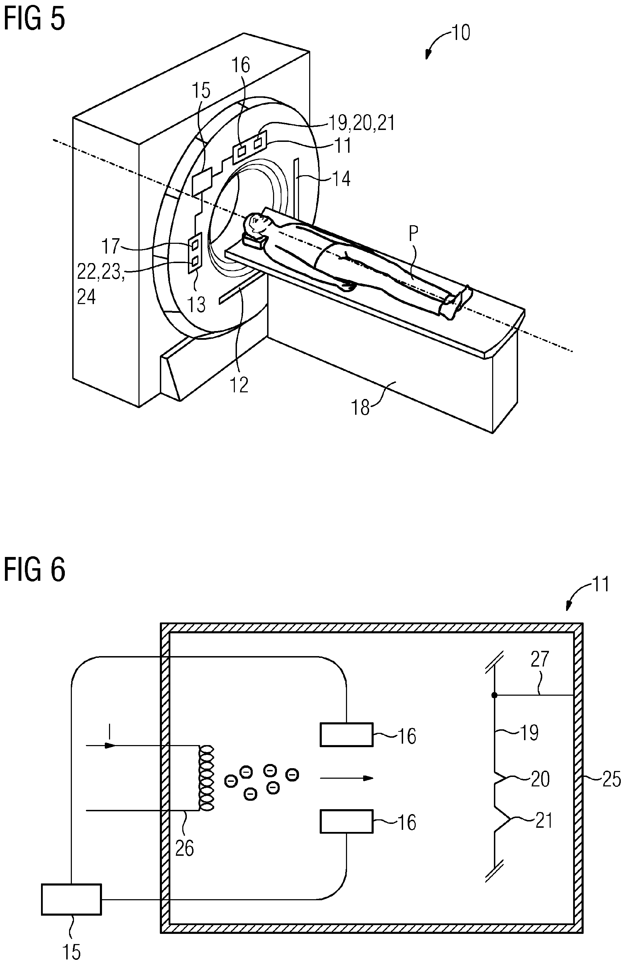

**Fig. 5** zeigt die Röntgeneinrichtung 10 in einer perspektivischen Ansicht. In diesem Ausführungsbeispiel ist die Röntgeneinrichtung 10 als Dual-Röntgenröhren-Computertomograph ausgebildet. Die Röntgeneinrichtung 10 weist eine Röntgenröhre 11 und einen Röntgendetektor 12 auf. Außerdem weist die Röntgeneinrichtung 10 eine weitere Röntgenröhre 13 und einen weiteren Röntgendetektor 14 auf. Die Röntgeneinrichtung 10 weist eine Steuereinheit 15 auf, welche mit einer Ablenkeinheit 16 der Röntgenröhre 11 und mit einer Ablenkeinheit 17 der weiteren Röntgenröhre 13 verbunden ist. Die Ablenkeinheit 16 und/oder die Ablenkeinheit 17 können jeweils als elektromagnetische Ablenkeinheit oder gemäß einem Wehneltzylinder ausgebildet sein.

Ein Patient P ist auf einer Patientenliege 18 während einer bildgebenden Messung gelagert.

Die Röntgenröhre 11 weist eine strukturierte Anode 19 mit einer ersten Anodenmikrostruktur 20 und einer zweiten Anodenmikrostruktur 21 auf. Die weitere Röntgenröhre 13 weist eine weitere strukturierte Anode 22 mit einer dritten Anodenmikrostruktur 23 und einer vierten Anodenmikrostruktur 24 auf.

Die Röntgenröhre 11 und die Röntgenröhre 13 werden im Gegentakt betrieben. Insbesondere wird zusätzlich zu den Verfahrensschritten gemäß dem in Fig. 1 gezeigten Ausführungsbeispiel die weitere strukturierte Anode 22 mit der dritten Anodenmikrostruktur 23 und der vierten Anodenmikrostruktur 24 als Teil der weiteren Röntgenröhre 13 bereitgestellt, wobei eine dritte Röntgenstrahlungsintensitätskennzahl gemäß der dritten Anodenmikrostruktur 23 und eine vierte Röntgenstrahlungsintensitätskennzahl gemäß der vierten Anodenmikrostruktur 24 in der Steuereinheit 15 bereitgestellt wird, wobei ein weiterer Sollwert einer Intensität einer Röntgenstrahlung der weiteren strukturierten Anode 22 in der Steuereinheit 15 komplementär zum ersten Sollwert ermittelt wird, wobei die dritte Röntgenstrahlungsintensitätskennzahl oder die vierte Röntgenstrahlungsintensitätskennzahl als weitere Sollwertröntgenstrahlungsintensitätskennzahl gemäß dem weiteren Sollwert der Intensität der Röntgenstrahlung in der Steuereinheit 15 ausgewählt wird und wobei ein weiterer Elektronenstrahl der weiteren Röntgenröhre 13 auf die dritte Anodenmikrostruktur 23 oder auf die vierte Anodenmikrostruktur 24 gemäß der ausgewählten weiteren Sollwertröntgenstrahlungsintensitätskennzahl zur Generierung der Röntgenstrahlung ausgerichtet wird, wodurch die Intensität der Röntgenstrahlung der weiteren strukturierten Anode 22 festgelegt wird.

**Fig. 6** zeigt die Röntgenröhre 11 in einem weiteren Ausführungsbeispiel in einem Querschnitt.

Die Röntgenröhre 11 weist ein Gehäuse 25 auf. Das Gehäuse 25 weist typischerweise einen Röngenstrahlungsdurchtrittsfenster auf, durch welches die mittels des auf der strukturierten Anode 19 auftreffenden Elektronenstrahls generierte Röntgenstrahlung vorzugsweise austreten kann. Die Röntgenstrahlung und das Röngenstrahlungsdurchtrittsfenster sind in Fig. 6 nicht gezeigt. Innerhalb des Gehäuses 25 ist ein Vakuum. Innerhalb des Gehäuses 25 ist eine Kathode 26 mit einem Emitter zur Emission des Elektronenstrahls mit einem Heizstrom I angeordnet. Zwischen der Kathode 26 und der strukturierten Anode 19 ist die Ablenkeinheit 16 angeordnet und eine Beschleunigungsspannung angelegt. Die Ablenkeinheit 16 kann die elektromagnetische Ablenkeinheit oder der Wehneltzylinder sein. In diesem Ausführungsbeispiel unterscheidet sich die erste Anodenmikrostruktur 20 von der zweiten Anodenmikrostruktur 21 in einer Rillenbreite und in einer Rillentiefe. Alternativ oder zusätzlich kann sich die erste Anodenmikrostruktur 20 von der zweiten Anodenmikrostruktur 21 in einem der folgenden Parameter unterscheiden:
- einem Mittelpunktabstand,
- in einer Punktierung einer Rille.

In diesem Ausführungsbeispiel ist die Steuereinheit 15 außerhalb des Gehäuses 25 angeordnet. Der Pfeil kennzeichnet die Elektronenstrahlrichtung. Die strukturierte Anode 19 weist eine Drehachse 27 auf und ist beispielsweise rotationssymmetrisch ausgebildet.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Festlegen einer Intensität einer Röntgenstrahlung mit einer strukturierten Anode (19), mit den folgenden Schritten:
- Bereitstellen einer Röntgenröhre (11), welche eine strukturierte Anode (19) mit einer ersten Anodenmikrostruktur (20) und einer zweiten Anodenmikrostruktur (21) aufweist,
- Bereitstellen einer ersten Röntgenstrahlungsintensitätskennzahl gemäß der ersten Anodenmikrostruktur (20) und einer zweiten Röntgenstrahlungsintensitätskennzahl gemäß der zweiten Anodenmikrostruktur (21) in einer Steuereinheit (15),
- Ermitteln eines ersten Sollwerts der Intensität der Röntgenstrahlung in der Steuereinheit (15) gemäß einem bei einer bildgebenden Messung zu untersuchenden Patienten (P),
- Auswählen der ersten Röntgenstrahlungsintensitätskennzahl oder der zweiten Röntgenstrahlungsintensitätskennzahl als erste Sollwertröntgenstrahlungsintensitätskennzahl gemäß dem ersten Sollwert der Intensität der Röntgenstrahlung in der Steuereinheit (15) und
- Ausrichten eines Elektronenstrahls der Röntgenröhre (11) auf die erste Anodenmikrostruktur (20) oder auf die zweite Anodenmikrostruktur (21) gemäß der ausgewählten ersten Sollwertröntgenstrahlungsintensitätskennzahl zur Generierung der Röntgenstrahlung, wodurch die Intensität der Röntgenstrahlung der strukturierten Anode (19) festgelegt wird.

2. Verfahren nach Anspruch 1, wobei ein zweiter Sollwert der Intensität der Röntgenstrahlung in der Steuereinheit (15) gemäß dem bei der bildgebenden Messung zu untersuchenden Patienten (P) ermittelt wird, wobei eine zweite Sollwertröntgenstrahlungsintensitätskennzahl gemäß dem zweiten Sollwert der Intensität der Röntgenstrahlung in der Steuereinheit (15) ausgewählt wird und wobei der Elektronenstrahl der Röntgenröhre (11) auf die erste Anodenmikrostruktur (20) oder auf die zweite Anodenmikrostruktur (21) gemäß der ausgewählten zweiten Sollwertröntgenstrahlungsintensitätskennzahl erneut ausgerichtet wird.

3. Verfahren nach Anspruch 2, wobei das erneute Ausrichten des Elektronenstrahls gemäß der ausgewählten zweiten Sollwertröntgenstrahlungsintensitätskennzahl innerhalb einer Elektronenstrahlausrichtungszeit von weniger als 1 s nach dem Ausrichten des Elektronenstrahls gemäß der ausgewählten ersten Sollwertröntgenstrahlungsintensitätskennzahl erfolgt.

4. Verfahren nach Anspruch 3, wobei die Elektronenstrahlausrichtungszeit geringer ist als 1 ms.

5. Verfahren nach einem der Ansprüche 3 bis 4, wobei die Elektronenstrahlausrichtungszeit auf eine Röntgendetektorauslesezeit der bildgebenden Messung abgestimmt wird.

6. Verfahren nach einem der vorhergehenden Ansprüche, wobei sich die erste Anodenmikrostruktur (20) von der zweiten Anodenmikrostruktur (21) in zumindest einem der folgenden Parameter unterscheidet:
- einer Rillentiefe,
- einer Rillenbreite,
- einem Mittelpunktabstand,
- in einer Punktierung einer Rille.

7. Verfahren nach einem der vorhergehenden Ansprüche, wobei der Elektronenstrahl mittels einer elektromagnetischen Ablenkeinheit oder mittels eines Wehneltzylinders ausgerichtet wird.

8. Verfahren nach einem der vorhergehenden Ansprüche, wobei eine weitere strukturierte Anode (22) mit einer dritten Anodenmikrostruktur (23) und einer vierten Anodenmikrostruktur (24) als Teil einer weiteren Röntgenröhre (13) bereitgestellt wird, wobei eine dritte Röntgenstrahlungsintensitätskennzahl gemäß der dritten Anodenmikrostruktur (23) und eine vierte Röntgenstrahlungsintensitätskennzahl gemäß der vierten Anodenmikrostruktur (24) in der Steuereinheit (15) bereitgestellt wird, wobei ein weiterer Sollwert einer Intensität einer Röntgenstrahlung der weiteren strukturierten Anode (22) in der Steuereinheit (15) komplementär zum ersten Sollwert ermittelt wird, wobei die dritte Röntgenstrahlungsintensitätskennzahl oder die vierte Röntgenstrahlungsintensitätskennzahl als weitere Sollwertröntgenstrahlungsintensitätskennzahl gemäß dem weiteren Sollwert der Intensität der Röntgenstrahlung in der Steuereinheit (15) ausgewählt wird und wobei ein weiterer Elektronenstrahl der weiteren Röntgenröhre (13) auf die dritte Anodenmikrostruktur (23) oder auf die vierte Anodenmikrostruktur (24) gemäß der ausgewählten weiteren Sollwertröntgenstrahlungsintensitätskennzahl zur Generierung der Röntgenstrahlung ausgerichtet wird, wodurch die Intensität der Röntgenstrahlung der weiteren strukturierten Anode (22) festgelegt wird.

9. Verfahren für ein Festlegen einer Intensität einer Röntgenstrahlung mit einer Feldemitterkathode, mit den folgenden Schritten:
- Bereitstellen einer Feldemitterkathode,
- Bereitstellen einer Röntgenstrahlungsintensitätskennlinie als Funktion einer Emitterspannung an der Feldemitterkathode in einer Steuereinheit,
- Ermitteln eines Sollwerts der Intensität der Röntgenstrahlung gemäß einem bei einer bildgebenden Messung zu untersuchenden Patienten in der Steuereinheit,
- Auswählen der Emitterspannung aus der Röntgenstrahlungsintensitätskennlinie gemäß dem Sollwert der Intensität der Röntgenstrahlung und
- Anlegen der ausgewählten Emitterspannung an der Feldemitterkathode, wodurch die Intensität der Röntgenstrahlung festgelegt wird.

10. Verfahren für ein Festlegen einer Intensität einer Röntgenstrahlung mit einem fingerförmigen Kathodenkopf, mit den folgenden Schritten:
- Bereitstellen eines mäanderförmigen Flachemitters mit einem fingerförmigen Kathodenkopf,
- Bereitstellen einer Röntgenstrahlungsintensitätskennlinie als Funktion einer Sperrspannung an dem fingerförmigen Kathodenkopf in einer Steuereinheit,
- Ermitteln eines Sollwerts der Intensität der Röntgenstrahlung gemäß einem bei einer bildgebenden Messung zu untersuchenden Patienten in der Steuereinheit,
- Auswählen der Sperrspannung aus der Röntgenstrahlungsintensitätskennlinie gemäß dem Sollwert der Intensität der Röntgenstrahlung und
- Anlegen der ausgewählten Sperrspannung an dem fingerförmigen Kathodenkopf, wodurch die Intensität der Röntgenstrahlung festgelegt wird.

11. Röntgeneinrichtung (10), aufweisend
- die Röntgenröhre (11),
- einen Röntgendetektor (12) und
- die Steuereinheit (15), wobei die Röntgeneinrichtung (10) gemäß dem Verfahren für das Festlegen der Intensität der Röntgenstrahlung nach einem der vorhergehenden Ansprüche ausgebildet ist.

12. Single-Röntgenröhren-Computertomograph, aufweisend
- die Röntgeneinrichtung (10) nach Anspruch 11.

13. Dual-Röntgenröhren-Computertomograph, aufweisend
- die Röntgeneinrichtung (10) nach Anspruch 11, wobei die Röntgeneinrichtung die weitere Röntgenröhre aufweist und gemäß dem Verfahren für das Festlegen der Intensität der Röntgenstrahlung nach Anspruch 8 ausgebildet ist.

14. Computerprogrammprodukt, welches direkt in einen Speicher einer Recheneinheit ladbar ist, mit Programmcodemitteln, um ein Verfahren nach einem der Ansprüche 1 bis 10 auszuführen, wenn das Computerprogrammprodukt in der Recheneinheit ausgeführt wird.
